Europäisches Patentamt

European Patent Office

Office européen des brevets

(11) Publication number: **0 077 312**
**B1**

# EUROPEAN PATENT SPECIFICATION

(45) Date of publication of patent specification: **21.01.87**

(51) Int. Cl.⁴: **A 61 M 27/00**, **A 61 B 17/34**

(21) Application number: **82850196.5**

(22) Date of filing: **11.10.82**

(54) Trocar.

(30) Priority: **14.10.81 SE 8106075**

(43) Date of publication of application:
**20.04.83 Bulletin 83/16**

(45) Publication of the grant of the patent:
**21.01.87 Bulletin 87/04**

(84) Designated Contracting States:
**AT BE CH DE FR GB IT LI LU NL SE**

(56) References cited:
**WO-A-79/00221**
**FR-A- 587 547**
**FR-A-2 073 688**
**GB-A- 185 052**
**GB-A-1 158 648**
**US-A-3 160 157**
**US-A-3 844 272**

(73) Proprietor: **Aktiebolaget Meteve**
**Humlegardsgatan 3**
**S-412 74 Göteborg (SE)**

(72) Inventor: **Ekbaldh, Fred Vage Gunnar**
**PL 178**
**S-430 40 Särö (SE)**
Inventor: **Tillander, Hans**
**Humlegardsgatan 3**
**S-412 74 Göteborg (SE)**

(74) Representative: **Inger, Lars Ulf Bosson**
**L + U INGER Patentbyra HB Garvaregatan 12**
**S-262 00 Ängelholm (SE)**

Courier Press, Leamington Spa, England.

Description

## Technical Field

The present invention relates to a trocar for making an incision in skin and bringing a tube through said skin and adjacent tissue comprising an elongate stiff body having a front end formed with a cutting edge and a rear end having an attaching portion for connection to a catheter tube.

The object of the present invention is to obtain a trocar by means of which a safe penetration of tissue can be made with a reduced risk of damage to patient and surgeon.

## Background Art

At operations and often in other circumstances there is a need to insert a catheter tube through the skin and/or other tissues. In such instances one usually connects the catheter tube to a strong, cylindrical, often somewhat bent needle, and using this needle as an aid the tube is drawn through the tissues. Such a needle is called a trocar, and can often also be provided with a mandrel to facilitate skin penetration. The front end of the trocar is usually formed with a very sharp facet by grinding, which facet is needed to penetrate the tissues. However, cylindrical trocars have been found to be difficult to hold particularly when the surgeon is wearing gloves, and thus slips are often made at penetration, whereby too often the trocar slides between the fingers in a manner causing cut injuries to the surgeon's fingers. The cut injuries are often of a serious character, and can mean a serious break in an intense surgery program, as is common at hospitals. Sliding means a risk at penetration of other tissue of the patient than the tissue intended, causing a delayed healing procedure, as well.

Such trocars form cylindrical wounds in the skin, which wounds have provided to be more difficult to heal than other wounds, and are therefore easily infected causing further discomfort to the patient as a consequence.

FR—A—2 073 688 discloses a trocar, comprising an elongate rigid body that can have an oval or eye-shaped cross-sectional configuration, having at its front end a cutting edge and having at its rear end a cylindrical attaching portion for connection to a catheter tube.

US—A—3 160 157 shows a surgical needle having on its curved body two longitudinal ribs, both formed by stamping a portion of the needle blank, which is circular in cross-section and swaged towards the front end.

GB—A—185 052 shows a needle which may be applicable for surgical purposes, the shank of which is formed of conventional steel wire of circular cross-section and provides two opposite flats extending towards the front end.

## Description of the Present Invention

It has now surprisingly been shown possible to be able to eliminate the aforementioned drawbacks and to obtain a trocar which gives more easily healable wounds, and thereby a reduced risk for infections but primarily the risk of slippage and uncontrollable cut injuries to the surgeon's hands is reduced. The present invention is thereby characterized in that the elongate solid body of the trocar comprises a flattened oblong unit having a front end provided with a front edge arranged at an angle to the longitudinal axis of the unit to form an oblique point, and two, substantially flat opposed elongate surfaces intermediate the ends of the trocar to be grasped by a user, said front edge being provided with at least one facet that extends from said point rearwardly along said edge to form a sharpened cutting edge and enables said cutting edge to split said skin in a fissure direction, whereby use of said edge produces a fissure-shaped incision, and whereby the flattened shape of said unit enables the trocar to be used with reduced risk of slippage.

According to a preferred embodiment of the invention the oblong unit is bent substantially at the middle to a predetermined angle.

According to a further preferred embodiment of the invention the rear part of the trocar is provided with a rearwardly increasing enlargement.

Further characteristics are given in the accompanying claims.

## Best Mode of Carrying Out the Invention

In the following the invention will be more closely described with reference to the attached drawings, wherein

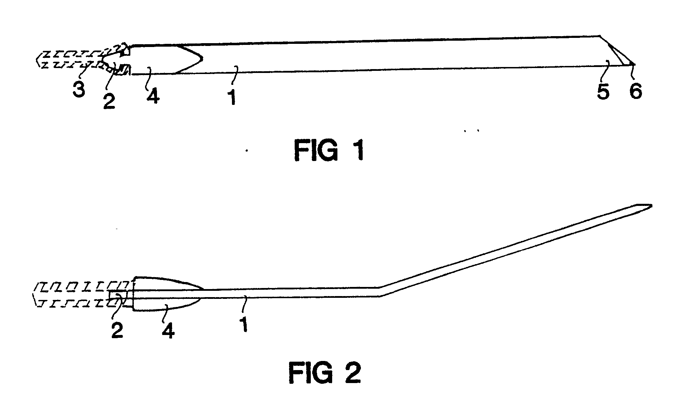

FIG. 1 shows a preferred embodiment of the invention seen from above;

FIG. 2 shows the embodiment of Fig. 1 seen from the side;

FIG. 3 shows another embodiment of the invention seen from above;

FIG. 4 shows the embodiment of Fig. 3 seen from the side;

FIG. 5 and 6 show different front end and edge embodiments; and

FIG. 7 shows another preferred embodiment of the rear end of the trocar.

1 denotes a flattened oblong unit suitably prepared in stainless steel. The length of the unit is about 15 cm and its width is half the circumference of a tube, or tubular means which is intended to be attached to the trocar, such a width being usually about 1 cm. The thickness of the unit is one to some millimeters, and must be enough to give a desired stiffness to the trocar. The thickness is thus dependent on the choice of material. The trocar 1 is bent, substantially at the middle, to an angle of about 20°. The trocar 1 is at its rear end provided with an attaching portion 2 provided with lateral projections over which a tube 3 is intended to be brought and removably attached thereto. The attaching portion 2 is an integral part of the rear end of the trocar 1 and is suitably punched out of said rear end. In the rear end of the trocar 1, immediately in front of the attaching portion 2 an enlargement 4 is arranged. The enlargement 4 decreases in thickness in the direction towards the front end of the trocar 1.

The object of the enlargement 2 is to widen the incision of a tissue to a diameter, which is the same as that of a tube which is to be brought through the incision, and which is to be attached to the trocar 1. Besides serving to widen the incision of a tissue the object of the enlargement 4 is to protect the tube 3 from being pushed off the attaching portion 2 and thereby be involuntarily removed from the trocar 1. The enlargement 4 can be said to be drop-shaped with a flattened thick-end. The trocar 1 is in its front end obliquely cut across its longitudinal axis to form an oblique tip with an asymmetrically placed point 5. The front end is formed, by means of grinding, with a cutting edge 6. The edge 6 extends from the tip at the point 5 across about two thirds of the width of the front end of the trocar 1.

The attaching portion 2 has a width which at least corresponds to half the circumference of the lumen of the tube, i.e., the diameter of the lumen is about a third of the width of the trocar 1. The attaching portion 2 is further somewhat tapered in order to facilitate the attachment of the tube 3.

The enlargement 4 is suitably a cast-on unit either or metal or of plastics.

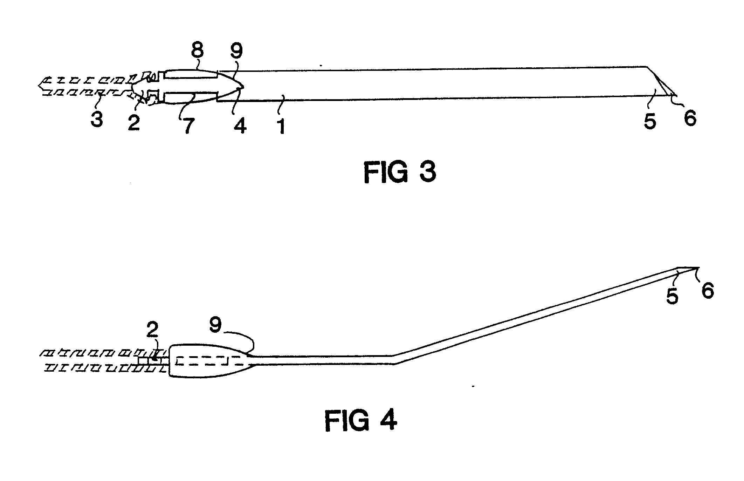

In Figs. 3 and 4 another preferred embodiment of the invention is shown, where the rear part of the trocar 1 is provided with an attaching portion 2 and in the front end of this there is a recess on each side, whereby the trocar 1 in this part has about half its width. The recesses are denoted 7. The enlargement 4 consists of an elastic unit 9 made out of a plastic material, which unit is provided with a through-bore which is threaded onto the trocar 1, the through-bore having a matching shape 8 that snaps into the recesses 7.

In using the trocar of the invention it has been found that lateral cutting effects are totally eliminated, whereby slipping and uncontrollable tissue penetration is avoided. Further the edge 6 shown enables the trocar 1 to split the skin in the fissure direction, whereby fissure shaped wounds are obtained, which heal rapidly without any complicating infection.

Thus it has been found that the trocar 1 can be easily handled, penetrates without difficulty, and gives exact holes through the skin, which holes hold the tube, very often a drainage tube, perfectly in place. Further, the trocar 1 penetrates the skin in such a way that, when extracting the tube, no painful widening occurs.

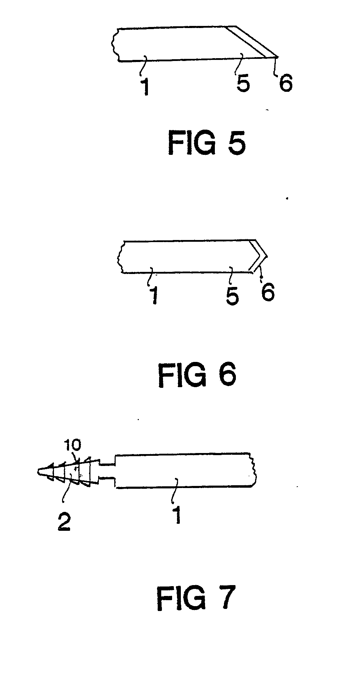

Although the front end and the edge 6 have been shown to be oblique with a two-third edge, the front end can be designed with an edge all over its entire length. Further, the front end can be designed with a more symmetric shape with a ground faceted edge as shown in Figs. 5 and 6.

The attaching portion 2 can further, as shown in Fig. 7 have a more extended shape with arranged counter-grips 10, which grip into the tube material and held the tube 3 to the trocar 1 during penetration.

Due to the simple technical shape of the trocar 1 it can be produced to a considerably lower price than trocars having a cylindrical shape. Depending on the low price the trocar can be produced as a disposable trocar, whereby expensive running sterilizations are eliminated.

The trocar 1 can be used for any type of tube, which needs to be brought through a tissue, such as catheters of different types, such as blood and urine catheters, or shunting tubes and stiff tubes for other liquids. Further the trocar can be used for drainage tubes, which are used to drain an operation area after a surgical incision in order to facilitate and speed up healing.

The tube 3 can hereby be attached to the trocar immediately prior to the penetration, or be delivered as a set together with the trocar, in the latter case the tube is suitably forced upon the attaching portion of the trocar by means of heat, and is removed by cutting the tube immediately adjacent the trocar.

The above trocar has been shown as a bent embodiment, wherein the bend has been formed substantially at the middle of the trocar. The bending can be carried out using another angle, greater or smaller, or can be displaced so that the front part becomes shorter or longer, or, in certain cases be completely eliminated, all depending on method or site of operation.

The material of the trocar has been said above to be stainless steel, but can of course be any other steel quality, which can be worked in the intended manner, e.g., to obtain a cutting edge. The trocar can further be made of a stiff plastic material, to the front end of which a steel edge has been attached.

## Claims

1. A trocar for making an incision in skin and bringing a tube through said skin and adjacent tissue comprising an elongate solid stiff body (1) having a front end (5) formed with a cutting edge (6) and a rear end having an attaching portion (2) for connection to a catheter tube (3) characterized in that the elongate solid body (1) comprises a flattened oblong unit having a front end (5) provided with a front edge (6) arranged at an angle to the longitudinal axis of the unit to form an oblique point, and two substantially flat opposed elongate surfaces intermediate said ends to be grasped by a user, said front edge (6) being provided with at least one facet that extends from said point rearwardly along said edge (6) to form a sharpened cutting edge (6) and enables said cutting edge (6) to split said skin in a fissure direction, whereby use of said edge (6) produces a fissure-shaped incision, and whereby the flattened shape of said unit (1) enables the trocar to be used with reduced risk of slippage.

2. A trocar according to claim 1, characterized in that said oblong unit (1) is bent substantially at the middle to a predetermined angle.

3. A trocar according to claim 2, characterized in that said predetermined angle is about twenty degrees.

4. A trocar according to claim 1, characterized in that said attaching portion (2) includes an enlargement means (4) that increases in thickness in a rearwardly direction for widening said incision in a lateral direction, thereby conforming the shape of incision substantially to that of the tube (3), and protecting the tube (3) from being involuntary pushed off during use of said trocar.

5. A trocar according to claim 1, characterized in that said facet extends rearwardly from said point along only about two-thirds of said front edge (6).

6. A trocar according to claim 1, characterized in that said oblique point of said front edge (6) is asymmetrically positioned to one side of said flattened unit (1).

## Patentansprüche

1. Trokar für die Erzeugung eines Einschnittes in Haut und das Einbringen einer Röhre durch diese Haut und benachbartes Gewebe mit einem länglichen, festen, starren Körper (1) mit einem mit einer Schneidkante (6) versehenen Vorderende (5) und einem mit einem Befestigungsabschnitt (2) zur Verbindung mit einer Katheterröhre (3) versehenen Hinterende, dadurch gekennzeichnet, daß der längliche feste Körper (1) eine abgeflachte längliche Einheit mit einem Vorderende (5), das mit einer in einem Winkel zu der Längsachse der Einheit unter Bildung eines Neigungspunktes angeordneten Vorderkante (6) versehen ist, und zwei im wesentlichen flachen, einander gegenüberliegenden länglichen Flächen zwischen den Enden, um von einem Benutzer ergriffen zu werden, versehen ist, wobei die Vorderkante (6) mit wenigstens einer Schliffffläche versehen ist, die sich von dem besagten Punkt nach hinten entlang seiner Kante (6) erstreckt, um eine geschärfte Schneidkante (6) zu bilden und die Schneidkante (6) in die Lage zu versetzen, die Haut in einer Spaltrichtung zu spalten, wodurch die Verwendung dieser Kante (6) einen spaltförmigen Einschnitt erzeugt, und wobei die abgeflachte Form der Einheit (1) der Trokar in die Lage versetzt, mit verminderter Abgleitgefahr verwendet zu werden.

2. Trokar nach Anspruch 1, dadurch gekennzeichnet, daß die längliche Einheit (1) im wesentlichen in der Mitte zu einem vorbestimmten Winkel abgebogen ist.

3. Trokar nach Anspruch 2, dadurch gekennzeichnet, daß der vorbestimmte Winkel etwa 20° beträgt.

4. Trokar nach Anspruch 1, dadurch gekennzeichnet, daß der Befestigungsabschnitt (2) eine Erweiterung (4) aufweist, deren Dicke in Richtung nach hinten zunimmt, um den Einschnitt in seitlicher Richtung aufzuweiten und so die Form des Einschnittes im wesentlichen in Übereinstimmung mit jener der Röhre (3) zu bringen und die Röhre (3) davor zu schützen, unfreiwillig während der Verwendung des Trokars abgestoßen zu werden.

5. Trokar nach Anspruch 1, dadurch gekennzeichnet, daß die Schliffffläche sich von dem besagten Punkt entlang nur etwa zwei Dritteln der Vorderkante (6) nach hinten erstreckt.

6. Trokar nach Anspruch 1, dadurch gekennzeichnet, daß der Neigungspunkt der Vorderkante (6) asymmetrisch zu einer Seite der abgeflachten Einheit (1) angeordnet ist.

## Revendications

1. Trocart destiné à pratiquer une incision dans la peau et à amener un tube à travers celle-ci et les tissus adjacents, comprenant un long corps rigide solide (1) présentant une extrémité avant (5) conformée avec un bord coupant (6), et un extrémité arrière présentant une partie de fixation (2) en vue d'une connexion au tube d'un cathéter (3), caractérisé en ce que le long corps solide (1) comprend une unité oblongue aplatie présentant une extrémité avant (5) comportant un bord avant (6) crée suivant un certain angle par rapport à l'axe longitudinal de l'unité en vue de former une pointe oblique, et deux longues surfaces opposées, essentiellement planes, intermédiaires aux extrémités susdites pour pouvoir être saisies par un usager, le bord avant susdit (6) comportant au moins une facette, qui s'étend vers l'arrière depuis la pointe le long de ce bord (6) en vue de former un bord coupant effilé (6), et qui permet à ce bord coupant (6) de fendre la peau dans la direction d'une fissure, de sorte que l'utilisation de ce bord (6) crée une incision en forme de fissure, et de manière que la forme aplatie de l'unité (1) permette l'utilisation du trocart avec un risque réduit de dérapage.

2. Trocart suivant la revendication 1, caractérisé en ce que l'unité oblongue (1) est coudée sensiblement en son centre suivant un angle prédéterminé.

3. Trocart suivant la revendication 2, caractérisé en ce que l'angle prédéterminée susdit est d'environ 20°.

4. Trocart suivant la revendication 1, caractérisé en ce que la partie de fixation (2) comprend un élément d'agrandissement (4) qui augmente d'épaisseur dans le sens arrière en vue d'élargir l'incision susdites en direction latérale, afin de conformer ainsi la forme de l'incision essentiellement à celle du tube (3) et de protéger ce tube (3) contre une poussée involontaire le séparant du trocart, durant l'utilisation de celui-ci.

5. Trocart suivant la revendication 1, caractérisé en ce que la facette susdite s'étend vers l'arrière depuis la pointe le long d'environ les deux tiers seulement du bord avant (6).

6. Trocart suivant la revendication 1, caractérisé en ce que la pointe oblique du bord avant (6) est prévue de manière asymétrique par rapport à un côté de l'unité aplatie (1).

**FIG 1**

**FIG 2**

FIG 3

FIG 4

FIG 5

FIG 6

FIG 7